# EUROPEAN PATENT SPECIFICATION

(11) **EP 1 741 410 B1**
(45) Date of publication and mention of the grant of the patent: **29.04.2009**
(21) Application number: 06116259.0
(22) Date of filing: 28.06.2006
(51) Int. Cl.: A61F 2/08, A61B 17/17

(54) **A jig for use with a ligament prosthesis**
Schablone zur Verwendung mit einem prothetischen Band
Gabarit pour utiliser avec un ligament prosthétique

(30) Priority: 04.07.2005 GB 0513686
(43) Date of publication of application: 10.01.2007
(73) Proprietor: Finsbury (Development) Limited, Leatherhead, Surrey KT22 0BA (GB)
(72) Inventor: Tuke, Michael Anthony, Guildford, Surrey GU1 3TF (GB)
(74) Representative: Smaggasgale, Gillian Helen

(56) References cited:
- EP-A- 0 413 549
- EP-A2- 1 302 167
- WO-A-20/04019824
- FR-A- 2 663 837
- US-A- 4 790 850
- US-A1- 2004 153 066
- US-A1- 2004 172 034
- US-A1- 2004 193 279

## Description

The present invention relates to a jig for use in locating the epicondylar axis which is useful in the placement of a replacement cruciate ligament. The replacement ligament may be a replacement anterior and/or posterior cruciate ligament in the knee, or a replacement collateral ligament for the knee. For ease of reference the replacement anterior and/or posterior cruciate ligament and the replacement collateral ligament will hereinafter be collectively referred to simply as the "replacement ligament". The present invention may be used in relation to a replacement cruciate ligament suitable for use with a prosthetic knee. According to a further aspect of the present invention there is provided a kit of components for use in the implantation of a replacement cruciate ligament.

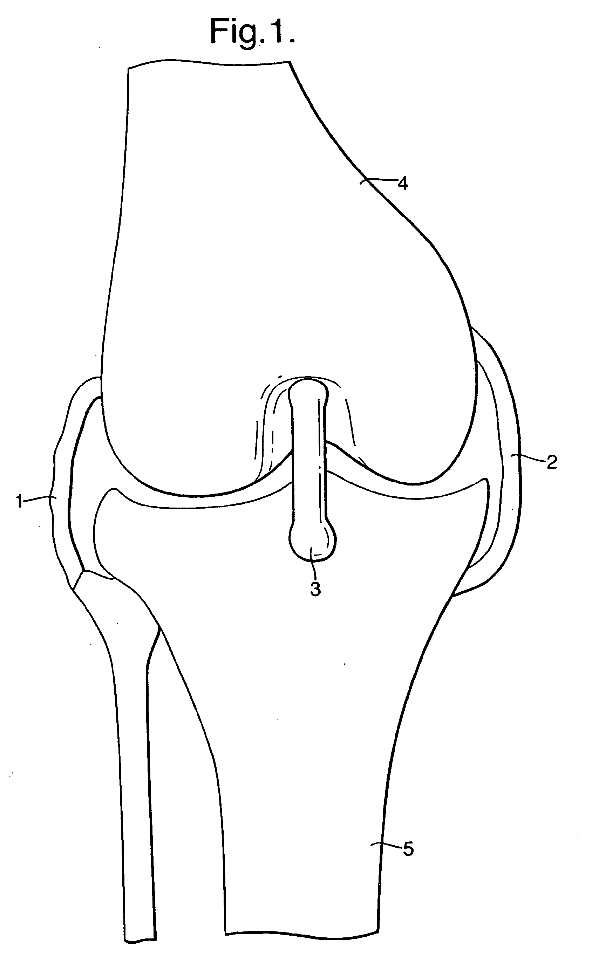

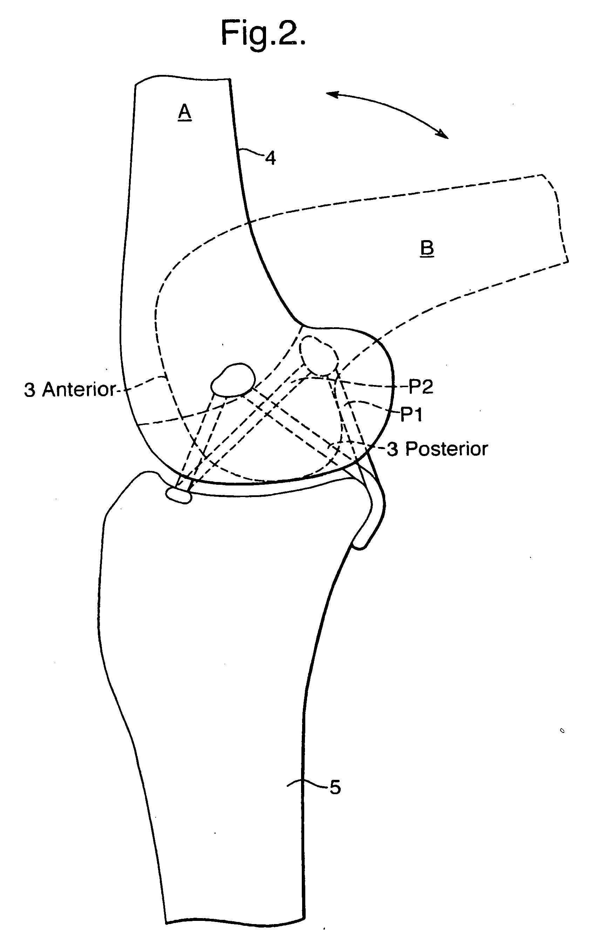

The human knee is a complex joint which is stabilised by several ligaments. The four most important of which are the lateral and medial collateral ligaments and the anterior and posterior cruciate ligaments. Figures 1 and 2 illustrate the position of the lateral collateral ligament 1, the medial collateral ligament 2 and the posterior and anterior cruciate ligaments 3. These ligaments each extend from the femur 4 to the tibia 5. The normal human knee relies heavily on the posterior and anterior cruciate ligaments 3 for anterior-posterior stability. This stability means that there is little or no freedom of motion other than hinge movement of the knee during flexion and extension. Whilst there is some rotation, there will be little or no anterior-posterior laxity. The position of the femur in extension (position A) and in flexion (position B) is illustrated in Figure 2. The location of the posterior and anterior cruciate ligaments in these positions is identified by dotted lines marked P1 and P2 respectively.

As the posterior and anterior cruciate ligaments are approximately centrally located in the medial and lateral direction in the knee, they allow controlled movement of the knee between the extension and flexion positions. It is also believed that the posterior and anterior cruciate ligaments work in conjunction with the medial collateral ligament in the overall anterior/posterior stability of the knee.

The posterior and/or anterior cruciate ligaments may become damaged through trauma, such as a sporting injury, or accident. The damage may be only a minor tear and may therefore in time heal. However, even if the tear heals successfully, the ligament may be too lax for correct functioning. Chronic cruciate ligament laxity or rupture may cause significant patella-femoral, and other knee function problems, due to instability of the femur on the tibia. More severe injuries can cause damage that will not heal to provide natural function and stability. Although ligament reconstructive techniques are performed relatively successfully in the unreplaced knee, they generally are difficult and may leave the patient in a worsening position than if there had been no treatment.

Where a patient has to undergo replacement knee surgery, the surgeon may retain and utilise the posterior cruciate ligament. However, it is rare for the anterior cruciate ligament to be retained for a total knee replacement. Further the retained posterior ligament is rarely of the the appropriate length to function correctly. It is therefore common for surgeons to remove all remnants of the anterior and posterior cruciate ligaments rather than retain them. One or both of the ligaments may also be removed in situations where the surgeon believes that retention will conflict with the motion of the replacement knee joint.

Prostheses for uni-compartmental or total knee replacement may have some laxity in flexion, i.e. anterior/posterior mechanical instability. This is usually due to the implant having no intrinsic anterior/posterior stability. Additionally or alternatively poor placement of the implants will result in laxity in either, or both of, extension and flexion. In an attempt to address the problems associated with these knee prostheses and surgical flaws, alternative prostheses have been developed which are said to provide "posterior stabilisation". These knee prostheses generally include an upstanding polyethylene post located on the upper surface of the tibial component on the knee prosthesis and a central cam located between the condyles on the femoral component of the prosthesis.

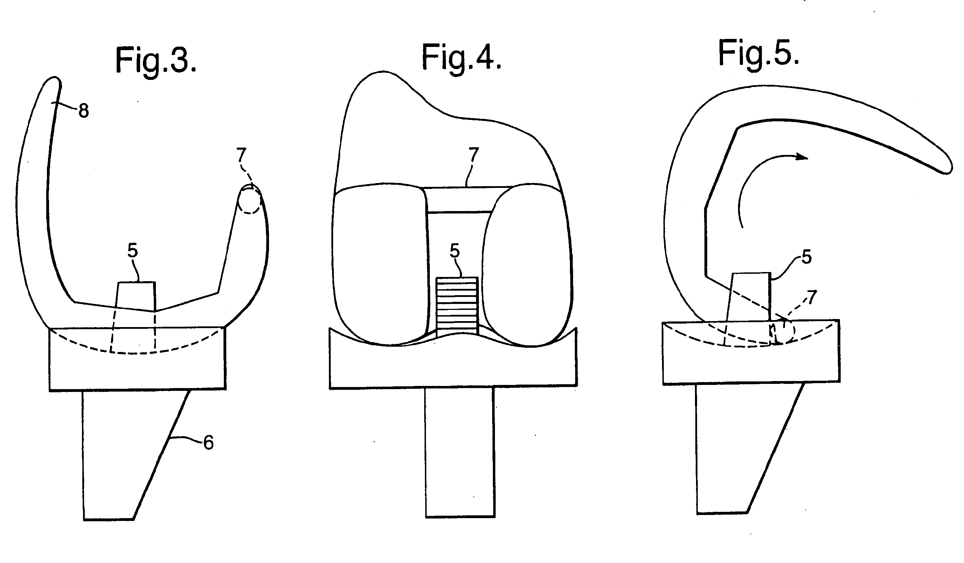

Figures 3 to 5 illustrate one example of a prosthesis of this type. The position of the post 5 on the tibial component 6 and its relationship with the cam 7 on the femoral component 8 are illustrated schematically in side view in Figure 3 and in front view in Figure 4. As the leg is moved, and the knee flexed, the femoral component 8 rotates until the cam 7 engages the post 5.

The interaction of the cam 7 with the post to prevent further rotation as illustrated in Figure 5. Once the cam 7 has engaged the post 5, any anterior movement by the femoral component on the tibial component is prevented. In some cases, the engagement with the post 5 forces a posterior motion of the femur on the tibia.

These prostheses are popular as they do provide some stability by enhancing the degree of flexion in lax knees by ensuring that the femur is placed relatively posteriorly on the tibia as the degree of flexion increases. However, the stability is only provided for a limited range of motion. In particular, the level of stability required for walking may not be provided. This is because the post and cam are not articulating together during the walking range of motion.

A further disadvantage of these prostheses is that the forces that the post and cam mechanism exert are not easy to predict or measure and are therefore not designed to resist for long implantation periods. This can lead to the parts wearing badly or even fracturing. This wear and stress can lead to the fixation between the implant and the bone being too high or changing, which in turn may lead to extra loosening loads being experienced. This in turn will lean to relatively early failure of the joint.

A still further disadvantage of posterior stabilised knee prostheses is that in order to insert the prostheses, the knee must be relatively lax. Thus the insertion of the knee prostheses may actually ensure or exacerbate the problems detailed above. Mobile tibia meniscus designs for knee replacements are now evolving which include such cam and post arrangements. However, these add a further engineering complexity that predisposes their failure.

Various attempts have been made to provide replacement ligaments for the normal or near-normal un-replaced knee. In one arrangement these replacement ligaments are natural ligaments harvested from another site in the patient. Whilst such ligaments may be useful, the harvesting step increases the cost of the operation and the suffering to the patient.

As an alternative to replacement natural ligaments for the normal knee artificial ligaments may be provided formed from plastics materials such as polyester. However, known artificial ligaments are generally unable to deal with the stresses and fatigue caused in the ligament at the femoral insertion point due to sustained repeated flexural motion.

Whilst replacement ligaments have offered some success with natural knee joints, artificial replacement ligaments have not generally been provided which can successfully be used with prosthetic knees.

The knee is expected to have a range of motion of up to about 150 degrees from full extension to full flexion. Any ligament must therefore be able to withstand this repeated angular displacement whilst under load. Most of the angular motion is concentrated at the insertion point of the ligament into the femur and hence the material properties are highly tested, man made fibres being vulnerable to flexural stress and fatigue failure. The placement of this insertion point is very critical for the knee to function correctly and to optimise the function of the ligament replaced. This placement is related to the articular surfaces of the femur and tibia which interact to provide instant centres of motion on the femur through which the ligament should be placed.

It is therefore desirable to provide replacement cruciate ligaments which can improve knee stability and reduce unwanted anterior-posterior movement of the femur when the knee is flexed. It is particularly desirable to provide replacement cruciate ligaments which may be successfully used with partial (uni-compartmental) or total prosthetic knee joints. It is further desirable to be able to avoid flexural failure and to place the femoral end of the ligament very accurately with respect to the articular surfaces of the knee throughout its motion. It is further desirable to provide replacement collateral ligaments.

It is also desirable that the replacement ligament be correctly aligned. US 2004/153066 describes a set of instruments and the method for use in preparing a knee joints to receive knee implants. Included in the description is a jig for use in locating the epicondylar axis which comprises a guide. Whilst this guide offers various advantages, it still suffers from various disadvantages and it is desirable to provide an improved means for locating an epicondylar axis. In addition, it is desirable to provide a jig which is easy to use.

The problems associated with prior art arrangements may be overcome by providing a jig for use in locating an epicondylar axis.

According to a first aspect of the present invention there is provided a jig for use in locating a selected condylar axis comprising:
two spaced rods extending radially from a guide; and
a finger extending from the distal end of each rod and being located substantially at right angles thereto such that the fingers are parallel and are adapted in use to be located against the femoral condyles of a flexed femur,
the rods being of the same length which is selected such that when the fingers are located against the femoral condyles, the guide is aligned approximately on the epicondylar axis or flexion axis of the knee.

Without wishing to be bound by any particular theory, there has been some discussion that the epicondylar axis and the flexion axis of the knee are not collinear. Discussions as to, if they are different, which is the correct one relevant to knee function are ongoing.

In use the jig will generally be located on the femur in maximum flexion and the fingers will sit on the femoral condyles to reference the distal and about 60° to about 90° degrees into flexion surfaces of the femur. By this means the convergence of the tangents to these two angular positions locates the centre point on the medial condyle. In a natural knee this point may be referred to as the medial epicondyle. Where a replacement knee is present, the point may be referred to as a new artificially created epicondyle based upon the new knee implant. The guide may also be used to pinpoint the lateral epicondyle.

The guide may be of any suitable shape but will generally be of substantially circular configuration. In one arrangement it may be formed as only part of a circle. In use the surgeon may use the guide to simply mark the position of the axis. However, in a preferred arrangement, the guide is a drill guide and therefore is configured to ensure that the drill is correctly lined up on the axis.

The rods may be of any suitable size. In one arrangement, the rods may be between about 15mm and about 30mm. The fingers may also be of any suitable size. A suitable size is generally about 60mm and about 120mm. Particularly suitable combinations are: rods of about 20mm in length with fingers of about 60mm in length; rods of about 24mm in length with fingers of about 70mm in length; or rods of about 26mm in length with fingers of about 80mm in length. In one alternative arrangement, the rods and/or the fingers may be adjustable in length. For example, they may be telescopic.

The jig may be formed of any suitable material although it will preferably be formed from metal.

In use the jig will generally be stabilised. Stabilisation may be provided by means of pins. In one arrangement, it may be clamped to at least one of the condyles, for example to either side of the femoral condyles.

According to a second aspect of the present invention there is provided a kit of parts comprising the jig of the above-mentioned first aspect and the components of a replacement ligament prosthesis wherein the replacement ligament prosthesis comprises:
an elongate replacement ligament having a femoral end and a tibial end;
a bar, which in use will extend between the medial and lateral condyles of a femur and across the intercondylar notch, and to which the femoral end of the elongate replacement ligament will be fixed; and
fixing means, which in use will fasten the tibial end of the elongate replacement ligament to the tibia.

The replacement ligament prosthesis may be used in connection with any non replaced knee with a deficient ligament or any type of surface replacing knee implant prostheses including partial and total knee replacement prostheses. Thus in the following discussion, reference to the femur or tibia and parts thereof may be natural components or prosthetic replacements therefor.

The elongate replacement ligament may be of any suitable configuration. In one arrangement, the replacement ligament may be formed from a plurality of fibres that may be combined to form the ligament in any suitable manner. In one arrangement, the fibres may be twisted together to form a rope-like structure.

The replacement ligament may be formed from any suitable material. In general, the material from which the replacement ligament is formed will have high strength in tension. Suitable materials include natural fibres such as muscle fascia, or synthetic fibres such as polyester, Dacron, nylon, carbon, Kevlar and the like. These materials may be used alone or in combination.

The replacement ligament may be of any suitable size. It may have a cross-sectional diameter of from about 2 to about 10 mm. In one arrangement, it may have a cross-sectional diameter of from about 4 to about 8 mm and preferably will be about 6 mm.

The length of the replacement ligament will be selected to be appropriate for the patient being treated. In general it will be from about 50 to about 250 mm in length. It is preferred that in use, the replacement ligament will pass through a bore in the tibia from the posterior surface to the anterior surface and will then be fixed at the anterior surface. It is therefore desirable that the replacement ligament will be of sufficient length to achieve this. However, it will generally be provided of longer length and then when fitted will be cut to the correct length.

As detailed above, in use, the replacement ligament may be passed through a bore in the tibia. To assist in this feeding, the tibial end of the elongate replacement ligament may be provided with a leader line. The leader line may be made of any suitable material. In an alternative arrangement, the leader line may be separate and in use passed through a folded portion of the replacement ligament to assist in feeding the ligament through a bore in the tibia. Suitable materials include natural fibres such as muscle fascia, or synthetic fibres such as polyester, Dacron, nylon, carbon, Kevlar and the like. Mixtures of these fibres may also be used. The cross-sectional diameter of the leader will generally be less than that of the elongate replacement ligament. Whilst any suitable diameter may be used, these will generally be in the range of from about 0.5 mm to about 3mm.

In use, the replacement ligament may be attached to the bar by any suitable means. In one arrangement, the femoral end of the replacement ligament may be provided with a loop which in use will be passed over the bar. The loop may be integral with the replacement ligament or may be provided as a separate component which is connected to the ligament. In one arrangement, where the replacement ligament is formed from a stranded material such as when it is a rope-like material, the loop may be formed by splicing the ligament. Where a loop is present, a central bush may be present.

Where a loop is present, it will be of any suitable size. In one arrangement, it may be from about 2 to about 10 mm in diameter. More preferably, it may have a diameter of from about 4 to about 8 mm, and most preferably, a diameter of about 6 mm.

One alternative means for attaching the replacement ligament to the bar is simply tying the femoral end of the replacement ligament to the bar.

In a further alternative arrangement the replacement ligament may be attached to the bar by means of a locking means provided on the replacement ligament which can engage the bar. Examples of suitable locking means include eyelets, clamps and the like.

In a still further alternative arrangement, the means for connecting the replacement ligament may be provided on the bar. For example, the bar may include a notch into which the ligament may be engaged. In another arrangement an aperture may be provided in the bar through which the ligament can be passed. In this latter arrangement the replacement ligament may be provided with a pivotable pin which can be threaded through the aperture and then pivoted to a position which will prevent the ligament from being pulled back through the aperture.

As in use the bar will be located at the end of the femur so that it extends between the medial and lateral condyles of the femur, it will be understood that it is an elongate component. It may be of any configuration but in one arrangement may have a circular cross-section. Alternative arrangements the shape of the bar in cross-section may differ along its length.

The bar may be formed of any suitable material. Suitable materials include metals, ceramics and plastics. However, metals are generally preferred. One suitable metal is cobalt chrome.

In one arrangement, the bar may be provided with anti-movement means which will prevent one or more of rotation, medial-lateral movement, and anterior-posterior movement, when located in the intercondylar notch.

The anti-movement means may be formed by any suitable arrangement. Where the movement to be prevented is rotation, the bar may be configured such that it has a cross-section which will prevent rotation once the bar is in situ. Thus, the bar may be of non-circular cross-section. In one arrangement, the bar may be of generally circular cross-section in a central region and of non-circular cross-section at the ends.

In one arrangement, the anti-movement means may be provided by means of one or more fins extending from the bar which in use engage with the bone or prosthesis.

The bar may be held in place by any suitable means. In one arrangement, brackets may be located on the inner wall of the condyles into which the ends of the bar may be located. In a preferred arrangement, one or both ends of the bar will be located in a bore in the respective condyles. The bores communicate with the intercondylar notch. One or both bores may extend through the condyle. In one arrangement, the bore may extend through one condyle so that the bar may be inserted through the bore, across the intercondylar notch and into a corresponding bore in the other condyle. The bore in the other condyle will preferably not extend completely through the condyle. In this preferred arrangement, the bar may be a screw. In one arrangement it will be a self-tapping screw such that it is not necessary to pre-form the bore in the other condyle.

Where the bar is formed from a screw, the threads of the screw may serve as the anti-movement fins.

The tibial end of the elongate replacement ligament may be fixed to the tibia by any suitable fixing means. In one arrangement, the tibial end may be connected to the tibia by a staple, nail or screw. Where the fixing means is a screw, it is preferably a non-sharp edged screw.

It will be understood that when in position, the portion of the bar lying in the intercondylar notch will preferably be fully exposed. Generally, the portion of the bar in the intercondylar notch will be free from anti-movement means. In a preferred arrangement this portion of the bar will be cylindrical and polished such as that in arrangements when a loop is present on the elongate replacement ligament, it can seat around the bar.

It will be understood that for the correct functioning of the replacement ligament, the bar is preferably located to lie on the centre of rotation of the knee femur as determined by the articular surfaces of the femur whether a natural or replacement knee femur. Where the bar is to be placed in a bore in the condyle, it may be desirable to utilise a jig to guide the axial direction of the drill which will be used to produce the bore into which the bar will be placed. However, in some circumstances it may be desirable for the surgeon to select a different position for the bar where that will provide improved functioning. For example, a planned eccentricity for the posterior cruciate ligament can induce so called "roll back" of the femur on the tibia and a planned eccentricity for the anterior cruciate ligament may induce "roll forwards".

A remote navigation system may be used prior to surgery to track the motion of the knee and determine the appropriate axis on which the bar should be placed.

The jig of the present invention will preferably allow the surgeon to provide the bore for the bar at a selected but defined position.

Where more than one ligament is to be replaced, they may be attached to the same bar or in one arrangement located at different bars located on different axis through the knee joint.

The kit may additionally include a partial or total replacement knee prosthesis.

The kit may additionally include a wire finder.

In order to introduce the replacement ligament, the knee, whether natural or prosthetic will be flexed to expose the femoral condyles. The epicondylar axis or the flexion axis may then be located utilising the jig of the present invention. Where the bar is to be inserted in a bore which extends through one condyle, a drill may be used to create a bore along the epicondylar axis or the flexion axis. It is likely that the drill will pass through the medial condyle, through the inter-condylar notch and into the lateral condyle. It will be understood that the drill could be operated from the lateral condyle side however, this will not generally be preferred.

The femoral bore may or may not pass through the entirety of the lateral condyle. The diameter of the bore will be selected to correspond to the bar which is to be introduced. In one arrangement, the drill bit may have steps of different diameter along its length or be performed in stages using different drill bits so that different diameters are obtained for different sections of the bone. The bar will then be introduced into the bore. Where the bar is a screw, it will be screwed into position.

Where the ligament is to be connected to the bar by means of a loop in the ligament, an eyelet or a boss, the bar will be passed through the loop or other means as it is passed through the intercondylar notch.

A tibial bore is produced which extends from the anterior surface of the tibia to the posterior surface of the tibia for the posterior ligament and similarly as appropriate and normally exercised for an anterior ligament. If both the anterior and posterior ligaments are being replaced the bar may accommodate both ligaments or their respective boss/eyelets. In one alternative arrangement the two ligaments may be connected before they are connected with the bar. In one arrangement, they may both surround a single eyelet or boss. The position where the tibial bore emerges on the surface of the tibia will be the position at which the ligament would naturally be joined to the tibia. The tibial bore may be produced by any suitable means. It will be understood that the bore may be produced by drilling in either direction.

The ligament member is passed through the tibial bore. This may be done using a wire finder. Before the end of the ligament member is fixed to the tibia, the surgeon will generally make an assessment of the optimum tension of the ligament member. This may involve taking up the slack in the ligament member and testing the knee in tension and flexion to determine the optimal length of the ligament member.

The ligament member is then fixed to the tibia using the fixing means. Fixing may be achieved by inserting the fixing means into the drilled hole such that the fixing means grips the ligament member tightly against the inner wall of the tibial bore. However, any means of permanently securing the tibial end of the ligament member to the tibia may be employed in the present invention.

It will be understood that the above steps may take place in any suitable order. For example the bones in the femur and tibia may both be formed before the components of the replacement ligament prosthesis are used.

Thus, the ligament prosthesis of the above first aspect of the invention can be implanted by means of a method which comprises:
i) locating an epicondylar axis;
ii) providing a femoral bore approximately on the epicondylar axis or flexion axis into the femur;
iii) making a tibial bore which extends from the anterior surface of the tibia to the posterior surface of the tibia;
iv) passing the ligament through the tibial bore;
v) locating the bar in the bore;
vi) coupling the bar and the elongate replacement ligament; and
vii) fixing the ligament to the tibia using fixing means.

The components of the ligament prosthesis are preferably those of the above first aspect.

The present invention will now be described, by way of example, with reference to the following examples in which:
- Figure 1: is an anterior-posterior view of the knee joint;
- Figure 2: is a medial-lateral view of the knee joint;
- Figure 3: is a schematic medial-lateral view of a knee prosthesis of the prior art in tension;
- Figure 5: is a anterior-posterior view of a knee prosthesis of the prior art in flexion;
- Figure 6: is the schematic illustration of the jig of the second aspect of the present invention;
- Figure 7: is a medial-lateral view of a knee in flexion with the jig in position;
- Figure 8: is an anterior-posterior view of Figure 7;
- Figure 9: is a medial-lateral view of a knee in flexion including a replacement posterior cruciate ligament of the present invention; and
- Figure 10: is an anterior-posterior view of Figure 9.

The present invention will now be described with reference to a knee prosthesis. However, it will be understood that it may be utilised with a normal knee.

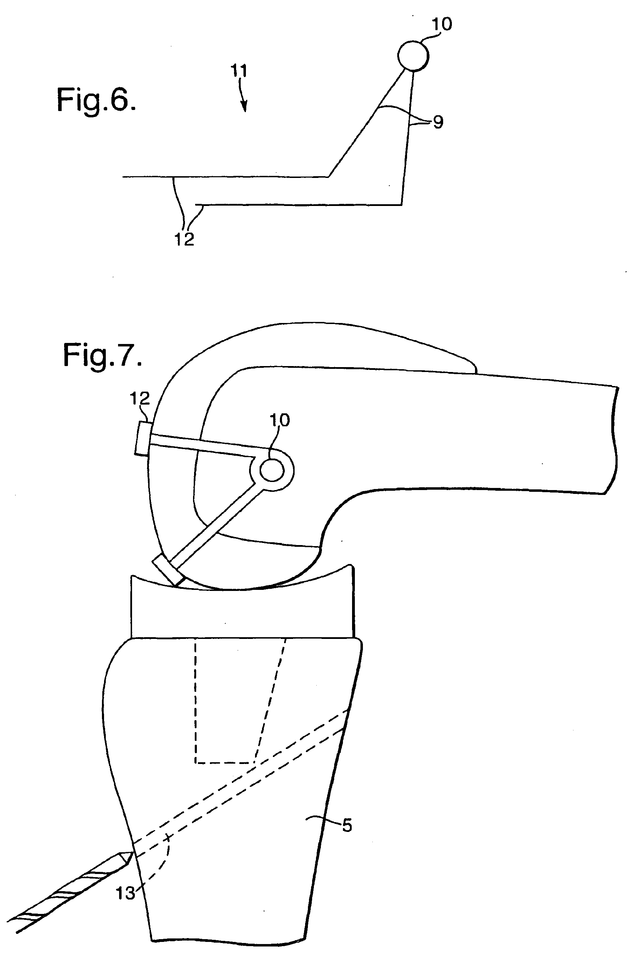

As illustrated in Figure 6 a jig 11 comprises rods 9 connected to a guide 10. These are angled such they represent the arms of a segment of a circle. The arms are in the same plane and are of the same length. Fingers 12 extend from the end of the arms.

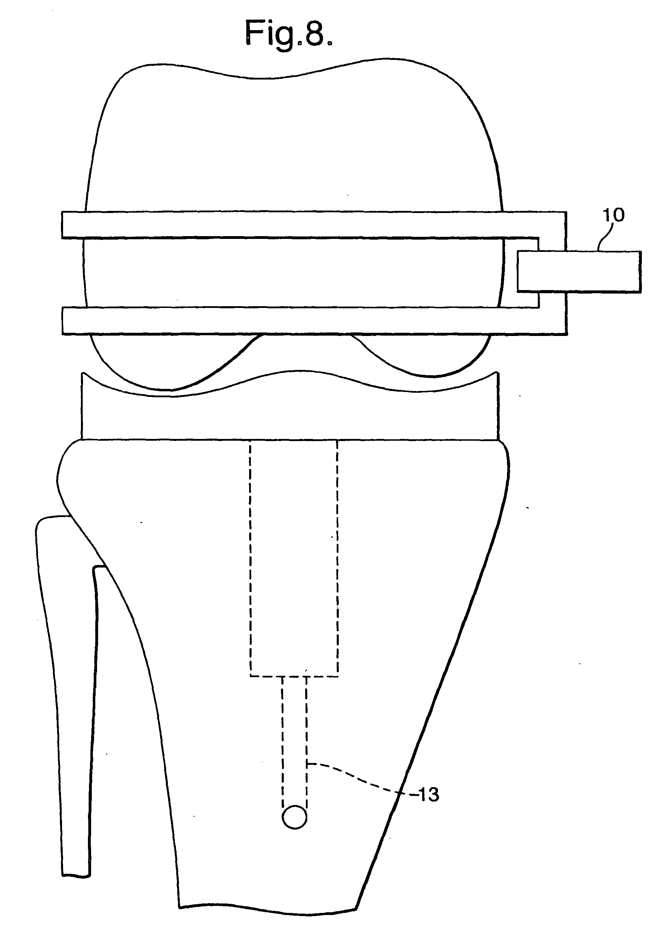

The jig 11 is then placed against the exposed knee which is positioned in flexion as illustrated in Figures 7 and 8. The fingers 12 are located against the face of the condyles with an upper finger being placed at the end of the femur 3. The aperture 10 lies on the epicondylar axis. As illustrated in Figure 8, the aperture may be a drill guide.

Once the drill guide 12 is correctly positioned, it may be stabilised using pins or may be clamped on either side of the inter-condylar notch or the femur. The femoral bore will then be made and the jig removed.

A tibial bore 13 is provided to extend from the anterior surface of the tibia 5 to the posterior surface thereof.

A femoral loop end of the replacement ligament 21 is passed through the bore 22 in the tibia using a wire finder (not shown).

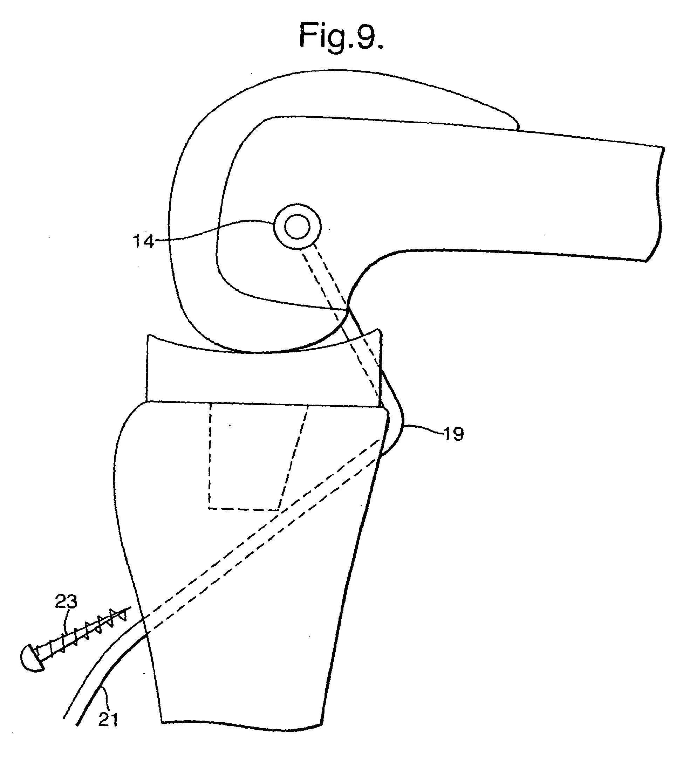

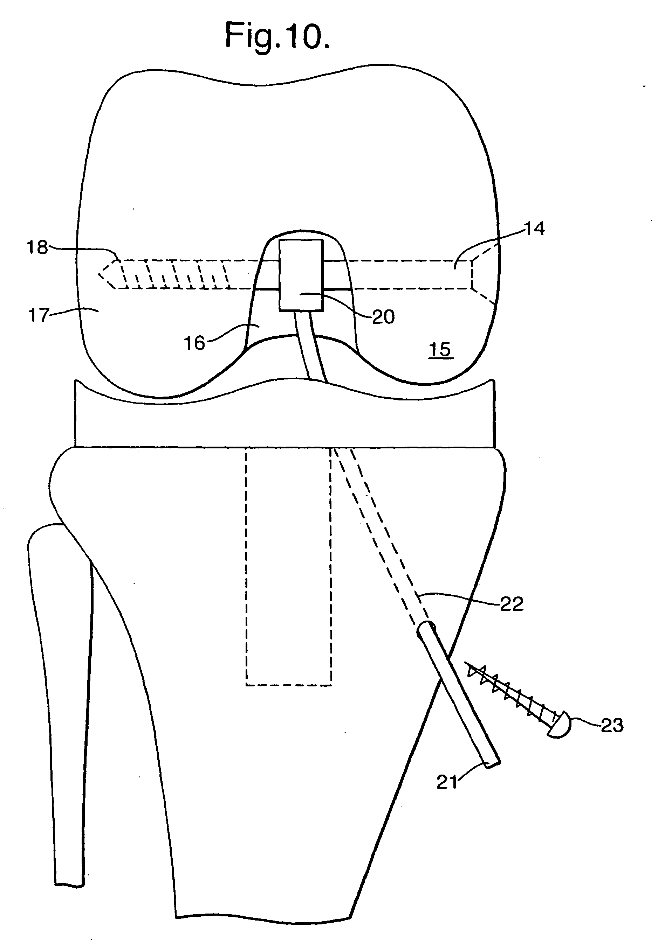

As illustrated in Figures 9 and 10, the bar 14 is inserted into the bore. The bar 14 is passed through the entirety of the medial condyle 15. The replacement ligament 19 has a loop 20 at the femoral end thereof through which the bar is passed and the bar then further inserted across the inter-condylar notch 16 and into the lateral condyle 17. The bar 14 includes fins provided by the screw-threads 18 on the bar to secure it in the bone of the femur.

The replacement ligament 19 has a loop 20 at the femoral end thereof through which the bar is passed.

Once the surgeon has made an assessment of the optimum tension for the replacement ligament, the ligament is fastened to the tibia using a screw 23. In one arrangement, the screw 23 is passed up the tibial bore 22 to fix ligament 21 in place. Any excess ligament 21 will be cut and removed.

## Claims

1. A jig (11) for use in locating the epicondylar axis comprising:
two spaced rods (9) extending radially from a guide (10); and
a finger (12) extending from the distal end of each rod and being located substantially at right angles thereto such that the fingers (12) are parallel and are adapted in use to be located against the femoral condyles of a flexed femur,
the rods (9) being of the same length which is selected such that when the fingers are located against the femoral condyles, the guide (10) being aligned approximately on the epicondylar axis or flexion axis of the knee.

2. A jig (11) according to Claim 1 wherein in use the fingers (12) will sit on the femoral condyles to reference the distal end about 60° to about 90° degrees into flexion surfaces of the femur.

3. A jig (11) according to Claim 1 or 2 wherein the guide (10) is a drill guide (12).

4. A kit of parts comprising the jig of any one of Claims 1 to 3 and the components of a replacement ligament prosthesis wherein the replacement ligament prosthesis comprises:
an elongate replacement ligament (21) having a femoral end and a tibial end;
a bar (14), which in use will extend between the medial and lateral condyles (15, 17) of a femur and across the intercondylar notch (16), and to which the femoral end of the elongate replacement ligament (21) will be fixed; and
fixing means (23), which in use will fasten the tibial end of the elongate replacement ligament to the tibia.

5. A kit according to Claim 1 wherein the elongate replacement ligament is formed from a plurality of fibres.

6. A kit according to Claim 5 wherein the fibres are twisted together to form a rope-like structure.

7. A kit according to Claim 4 to 6 wherein the replacement ligament has a cross-sectional diameter of from 2 to 10 mm.

8. A kit according to any one of Claims 4 to 7 wherein the replacement ligament has a length of from 50 to 250 mm in length.

9. A kit according to any one of Claims 4 to 8 wherein the elongate replacement ligament is provided with a leader line.

10. A kit according to any one of Claims 4 to 9 wherein the femoral end of the replacement ligament is provided with a loop (20) which in use will be passed over the bar (14).

11. A kit according to Claim 10 wherein the loop (20) is integral with the replacement ligament.

12. A kit according to Claim 11 wherein the loop (20) is formed by splicing the replacement ligament.

13. A kit according to Claim 12 wherein a central bush is present.

14. A kit ligament according to any one of Claims 10 to 13 wherein the loop is from 2 to 10 mm in diameter.

15. A kit according to any one of Claims 4 to 14 wherein the bar (14) is provided with anti-movement means which in use prevents one or more of rotation, medial-lateral movement, and anterior-posterior movement, when located in the intercondylar notch (16).

16. A kit according to Claim 15 wherein the anti-movement means is provided by means of one or more fins extending from the bar (14) which in use engage with the bone or prosthesis.

17. A kit according to Claim 16 wherein the bar (14) is formed from a screw and the threads of the screw serve as the anti-movement fins.

18. A kit according to any one of Claims 4 to 17 wherein the fixing means is a staple, nail or screw (23).

19. A kit according to any one of Claims 4 to 18 wherein the portion of the bar (16) which will be located in the intercondylar notch (16) is cylindrical and polished.

20. A kit according to any one of Claims 4 to 19 wherein the kit additionally includes a partial or total replacement knee prosthesis.

21. A kit according to any one of Claims 4 to 20 wherein the kit additionally includes a wire finder.

## Patentansprüche

1. Schablone (11) zur Benutzung bei der örtlichen Festlegung der Epikondylenachse mit
zwei beabstandeten, von einer Führung (10) radial ausgehenden Stäben (9) und
einem Finger (12), der von dem distalen Ende jedes Stabes ausgeht und zu diesem im wesentlichen unter rechten Winkeln angeordnet ist, so daß die Finger (12) parallel sind und so eingerichtet sind, daß sie bei Benutzung an den Femurkondylen eines gebeugten Femurs liegen,
wobei die Stäbe (9) von der gleichen Länge sind, die so gewählt ist, daß die Führung (10) beim Anliegen der Finger an den Femurkondylen annähernd auf die Epikondylenachse oder Beugungsachse des Knies ausgerichtet ist.

2. Schablone (11) nach Anspruch 1, bei der bei Benutzung die Finger (12) auf den Femurkondylen aufsitzen, um das distale Ende etwa 60° bis etwa 90° in die Beugungsflächen des Femurs zu verweisen.

3. Schablone (11) nach Anspruch 1 oder 2, bei der die Führung (10) eine Rohrführung (12) ist.

4. Satz von Teilen mit der Schablone eines der Ansprüche 1 bis 3 und den Bestandteilen einer Ersatzbandprothese, wobei die Ersatzbandprothese umfaßt:
ein längliches Ersatzband (21) mit einem Femurende und einem Tibiaende,
einen Riegel (14), der sich bei der Benutzung zwischen den innern und seitlichen Kondylen (15,17) eines Femurs und quer zu der Interkondylargrube (16) erstreckt und an dem das Femurende des länglichen Ersatzbandes (21) befestigt wird, und
Befestigungsmittel (23), die bei der Benutzung das Tibiaende des länglichen Ersatzbandes an der Tibia befestigen.

5. Satz nach Anspruch 1, bei dem das längliche Ersatzband aus einer Mehrzahl von Fasern gebildet ist.

6. Satz nach Anspruch 5, bei dem die Fasern unter Bildung einer seilartigen Struktur verdrillt sind.

7. Satz nach Anspruch 4 bis 6, bei dem das Ersatzband einen Querschnittsdurchmesser von 2 bis 10 mm hat.

8. Satz nach einem der Ansprüche 4 bis 7, bei dem das Ersatzband eine Länge von 50 bis 250 mm hat.

9. Satz nach einem der Ansprüche 4 bis 8, bei dem das längliche Ersatzband mit einer Sehnenschnur versehen ist.

10. Satz nach einem der Ansprüche 4 bis 9, bei dem das Femurende des Ersatzbandes mit einer Schlaufe (20) versehen ist, die bei der Benutzung über den Riegel (14) gezogen wird.

11. Satz nach Anspruch 10, bei dem die Schlaufe (20) integraler Teil des Ersatzbandes ist.

12. Satz nach Anspruch 11, bei dem die Schlaufe (20) durch Spleißen des Ersatzbandes gebildet ist.

13. Satz nach Anspruch 12, bei dem eine zentrale Hülse vorhanden ist.

14. Satzband nach einem der Ansprüche 10 bis 13, bei dem die Schlaufe 2 bis 10 mm im Durchmesser ist.

15. Satz nach einem der Ansprüche 4 bis 14, bei dem der Riegel (14) mit einem Antibewegungsmittel versehen ist, das bei Benutzung bei Anordnung in der Interkondylargrube (16) eine oder mehrere Drehungen, mittlere-seitliche Bewegung und Vor-Zurück-Bewegung verhindert.

16. Satz nach Anspruch 15, bei dem das Antibewegungsmittel durch eine oder mehrere von dem Riegel (14) ausgehende Rippen gebildet ist, die bei Benutzung mit dem Knochen oder der Prothese in Eingriff sind.

17. Satz nach Anspruch 16, bei dem der Riegel (14) von einer Schraube gebildet ist und die Gewindegänge der Schraube als die Antibewegungsrippen dienen.

18. Satz nach einem der Ansprüche 4 bis 17, bei dem das Befestigungsmittel eine Krampe, ein Nagel oder eine Schraube (23) ist.

19. Satz nach einem der Ansprüche 4 bis 18, bei dem der Teil des Riegels (16), der in der Interkondylargrube (16) angeordnet ist, zylindrisch und poliert ist.

20. Satz nach einem der Ansprüche 4 bis 19, bei dem der Satz ferner eine Knieersatzteilprothese oder -gesamtprothese umfaßt.

21. Satz nach einem der Ansprüche 4 bis 20, bei dem der Satz ferner einen Drahtsucher umfaßt.

## Revendications

1. Gabarit (11) destiné à être utilisé dans la localisation de l'axe épycondylaire comprenant:
deux tiges espacées (9) s'étendant radialement depuis un guide (10) ; et
un doigt (12) s'étendant depuis l'extrémité distale de chaque tige et étant situé sensiblement en angle droit avec celle-ci de telle sorte que les doigts (12) soient parallèles et adaptés lors de l'utilisation pour être placés contre les condyles fémoraux d'un fémur fléchi,
les tiges (9) étant de la même longueur, laquelle étant sélectionnée de telle sorte que lorsque les doigts sont placés contre les condyles fémoraux, le guide (10) soit aligné approximativement sur l'axe épicondylaire ou axe de flexion du genou.

2. Gabarit (11) selon la revendication 1, dans lequel durant l'utilisation les doigts (12) reposent sur les condyles fémoraux afin de référencer l'extrémité distale à environ 60° à environ 90° dans les surfaces de flexion du fémur.

3. Gabarit (11) selon la revendication 1 ou 2, dans lequel le guide (10) est un guide pour perçeuse (12).

4. Kit de pièces comprenant le gabarit selon l'une quelconque des revendications 1 à 3 et les composants d'une prothèse de ligament de remplacement, dans lequel la prothèse de ligament de remplacement comprend :
un ligament de remplacement allongé (21) ayant une extrémité fémorale et une extrémité tibiale ;
une barre (14), qui durant l'utilisation s'étendra entre les condyles interne et externe (15, 17) d'un fémur et à travers l'échancrure intercondylaire (16), et à laquelle l'extrémité fémorale du ligament de remplacement allongé (21) sera fixée ; et
un moyen de fixation (23), qui durant l'utilisation fixera l'extrémité tibiale du ligament de remplacement allongé au tibia.

5. Kit selon la revendication 1, dans lequel le ligament de remplacement allongé est formé à partir d'une pluralité de fibres.

6. Kit selon la revendication 5, dans lequel les fibres sont torsadées ensemble afin de former une structure du type corde.

7. Kit selon les revendications 4 à 6, dans lequel le ligament de remplacement a un diamètre en coupe de 2 à 10 mm.

8. Kit selon l'une quelconque des revendications 4 à 7, dans lequel le ligament de remplacement a une longueur de 50 à 250 mm.

9. Kit selon l'une quelconque des revendications 4 à 8, dans lequel le ligament de remplacement allongé est muni d'une ligne de repère.

10. Kit selon l'une quelconque des revendications 4 à 9, dans lequel l'extrémité fémorale du ligament de remplacement est munie d'une boucle (20) qui durant l'utilisation sera passée par-dessus la barre (14).

11. Kit selon la revendication 10, dans lequel la boucle (20) fait partie intégrante du ligament de remplacement.

12. Kit selon la revendication 11, dans lequel la boucle (20) est formée en épissurant le ligament de remplacement.

13. Kit selon la revendication 12, dans lequel une bague centrale est présente.

14. Ligament en kit selon l'une quelconque des revendications 10 à 13, dans lequel la boucle a un diamètre de 2 à 10 mm.

15. Kit selon l'une quelconque des revendications 4 à 14, dans lequel la barre (14) est munie d'un moyen anti-mouvement qui durant l'utilisation empêche l'un ou plusieurs d'un mouvement de rotation, d'un mouvement médial-latéral, et d'un mouvement antérieur-postérieur, quand elle est située dans l'échancrure intercondylaire (16).

16. Kit selon la revendication 15, dans lequel le moyen anti-mouvement est constitué par une ou plusieurs ailettes qui s'étendant depuis la barre (14) et qui durant l'utilisation s'engagent avec l'os ou la prothèse.

17. Kit selon la revendication 16, dans lequel la barre (14) est formée par une vis et les filets de la vis servent d'ailettes anti-mouvement.

18. Kit selon l'une quelconque des revendications 4 à 17, dans lequel le moyen de fixation est une agrafe, un clou ou une vis (23).

19. Kit selon l'une quelconque des revendications 4 à 18, dans lequel la partie de la barre (16) qui sera placée dans l'échancrure intercondylaire (16) est cylindrique et polie.

20. Kit selon l'une quelconque des revendications 4 à 19, dans lequel le kit comporte de plus une prothèse de genou de remplacement partielle ou totale.

21. Kit selon l'une quelconque des revendications 4 à 20, dans lequel le kit comporte de plus un détecteur de fil métallique.
